# EUROPÄISCHE PATENTANMELDUNG

(11) **EP 4 520 353 A1**
(43) Veröffentlichungstag der Anmeldung: **12.03.2025**
(21) Anmeldenummer: 23196306.7
(22) Anmeldetag: 08.09.2023
(51) Int. Cl.: A61L 2/14

(54) **VERFAHREN UND VORRICHTUNG ZUR SCHAFFUNG EINER RNASE-FREIEN UND/ODER DNASE-FREIEN OBERFLÄCHE**

(71) Anmelder: 3CON Anlagenbau GmbH, 6341 Ebbs (AT); ionOXess GmbH, 6020 Innsbruck (AT)
(72) Erfinder: De Benedictis, Lorenzo, 6130 Schwaz (AT); Rupprich, Marco, 6094 Axams (AT); Obholzer, Thomas, 6080 Vill (AT)
(74) Vertreter: Schwarz & Partner Patentanwälte GmbH

(57) **Zusammenfassung**

Die vorliegende Erfindung betrifft ein Verfahren und eine Vorrichtung zur Schaffung einer RNase-freien und/oder DNase-freien Oberfläche, insbesondere einer RNase-freien und/oder DNase-freien Polymeroberfläche. Das Verfahren umfasst das Erzeugen eines ozon-dominierten nicht thermischen Plasmas unter atmosphärischem Druck und das Erzeugen eines stickstoff-dominierten nicht thermischen Plasmas unter atmosphärischem Druck. Das Verfahren umfasst ferner das Erzeugen einer Plasmaströmung, aus dem ozon-dominierten nicht thermischen Plasma und dem stickstoff-dominierten nicht thermischen Plasma und das Leiten der Plasmaströmung auf eine zu reinigende Oberfläche, sowie das Inaktivieren von RNase und/oder DNase auf der zu reinigenden Oberfläche.

## Beschreibung

### Gebiet der Erfindung

Die vorliegende Erfindung betrifft ein Verfahren und eine Vorrichtung zur Schaffung einer RNase-freien und/oder DNase-freien Oberfläche, insbesondere einer RNase-freien und/oder DNase-freien Polymeroberfläche.

### Hintergrund

RNA (Ribonucleic Acid) und DNA (Deoxyribonucleic Acid) sind zwei wichtige Nukleinsäuren, die in lebenden Organismen unterschiedliche Funktionen erfüllen. Wie allgemein bekannt ist, trägt die DNA die genetische Information, welche u.a. die Entwicklung, das Wachstum, die Funktion und die Vermehrung eines Organismus beeinflusst. Die DNA wird auf die nachfolgende Generation vererbt. Die RNA ist maßgeblich an der Proteinbiosynthese beteiligt. Die sogenannte messenger RNA (mRNA) überträgt die genetischen Informationen aus der DNA im Zellkern zu den Ribosomen im Zytoplasma, wo Proteine hergestellt werden.

Basierend auf RNA und/oder DNA werden derzeit neuartige Impfstoffe und medizinische Behandlungsmethoden entwickelt. Beispielsweise wurden sogenannte mRNA-Impfstoffe erfolgreich als COVID-19-Impfstoff eingesetzt. Weitere mRNA-Impfstoffe gegen Krankheiten wie beispielsweise Boreillose, Chlamydien, Cytomegalie, Dengue, Herpes, Grippe, HIV und Malaria (um nur einige zu nennen) sind derzeit in Entwicklung.

Zudem werden RNA und/oder DNA basierte Wirkstoffe auch in der Krebstherapie, insbesondere der Krebsimmuntherapie und zur Behandlung von genetischen Defekten erforscht. Diese RNA und/oder DNA basierten Technologien haben ein hohes Potential, derzeit nicht oder nur ungenügend behandelbare Krankheiten erfolgreich behandeln oder jedenfalls deren Symptome begrenzen zu können.

Allerdings führen schon kleine Verunreinigungen mit RNase (Ribonuklease) und/oder DNase (Desoxyribonuklease) dazu, dass die neuartigen Impf- und/oder Wirkstoffe beschädigt oder sogar unbrauchbar werden. Denn RNasen sind Enzyme, die die hydrolytische Spaltung von Phosphodiesterbindungen in Ribonukleinsäure(RNA)-Ketten katalysieren. Desoxyribonuklease ist ein Enzym, das die Hydrolyse von Desoxyribonukleinsäure-Molekülketten (DNA) in kürzere Molekülketten oder die Einzelbausteine katalysiert. Kommt es zu dieser Katalyse, kann der Impf- und/oder Wirkstoffe beschädigt, inaktiviert oder sogar unbrauchbar werden.

Daher müssen alle Behälter und sonstige Gegenstände, die mit den Impf- und/oder Wirkstoffen in Kontakt kommen (beispielsweise bei der Herstellung, der Lagerung, oder der Verabreichung) aufwändig gereinigt und von aktiven RNasen/DNasen befreit werden.

Diese Reinigung umfasst beispielsweise das Autoklavieren, die Behandlung mit Wasserstoffperoxid, mit Diethylpyrocarbonat oder mit Ribonuklease-Inhibitoren, die Behandlung mit Hitze, oder UV-Strahlung und/oder sonstige Verfahren.

Diese Reinigungsverfahren sind oft teuer, zeitaufwändig und können u.a. auch die behandelten Oberflächen bzw. Gegenstände nachhaltig schädigen. Beispielsweise ist es bekannt, dass RNasen und DNasen durch 15-minütiges Autoklavieren bei zumindest 121°C oder durch Erhitzen auf 180°C für zumindest acht Stunden zerstört werden. Die hohe Temperatur und/oder die eingesetzten Chemikalien beschädigen viele Werkstoffe, insbesondere Polymere, nachhaltig, sodass diese Werkstoffe nicht oder nur beschränkt für die Herstellung und Lagerung der neuen Impf- und Wirkstoffe eingesetzt werden können.

In herkömmlichen Bioreaktoren werden die herzustellenden Impf- und/oder Wirkstoffe üblicherweise in Edelstahltanks gelagert, die mit einer Polymerfolie oder einem Polymerfolienbeutel ausgekleidet sind und/oder über Polymerschläuche verbunden sind.

Typische Polymere für die Verwendung in Bioreaktoren sind
- ETFE - Ethylen-Tetrafluorethylen-Copolymer,
- FEP - Tetrafluorethylen-Hexafluorpropylen-Copolymer,
- HDPE - High Density Polyethylen,
- LDPE - Low Density Polyethylen,
- PC - Polycarbonate Polyethylenterephthalat,
- PFA - Perfluoralkoxy-Polymere,
- PP/PCO - Polypropylene/Polycyclooctene,
- PMP - Polymethylpenten,
- Barex - Acrylnitril-Kunststoff,
- HIPS - High Impact Polystyrene,
- PVC - Polyvinylchlorid,
- TPE - Theroplastisches Elastomer,
- und/oder dergleichen.

Diese sind jedoch nicht oder nur bedingt dazu geeignet mit herkömmlichen Verfahren von aktiver DNase und RNase befreit zu werden.

### Beschreibung der Erfindung

Es ist daher die Aufgabe der vorliegenden Erfindung ein Verfahren und eine Vorrichtung zur Schaffung einer RNase-freien und/oder DNase-freien Oberfläche bereitzustellen, welches Verfahren insbesondere auf Polymeroberflächen anwendbar ist. Diese Aufgabe wird durch ein Verfahren gemäß Anspruch 1 und eine Vorrichtung gemäß Anspruch 10 gelöst. Weitere Aspekte der Erfindung sind in den Unteransprüchen sowie der nachfolgenden Beschreibung angeführt.

Insbesondere wird die Aufgabe durch ein Verfahren zur Schaffung einer RNase-freien und/oder DNase-freien Oberfläche, insbesondere einer RNase-freien und/oder DNase-freien Polymeroberfläche gelöst. Der Begriff RNase-freie und/oder DNase-freie Oberfläche bezeichnet eine Oberfläche, die im Wesentlichen frei von aktiven RNasen und/oder DNasen ist. Eine im Wesentlichen RNasen und/oder DNasen freie Oberfläche ist eine Oberfläche, deren RNase und/oder DNase Kontamination so gering ist, dass RNA bzw. DNA haltige Produkte (wie Wirk- oder Impfstoffe), die mit dieser Oberfläche in Kontakt kommt nicht mehr beschädigt bzw. inaktiviert werden.

Das Verfahren ist auf alle Arten von Oberflächen anwendbar, insbesondere auf Polymeroberflächen. Es versteht sich jedoch, dass auch andere Oberflächen, wie beispielsweise metallische Oberflächen, keramische Oberflächen, Glasoberflächen und/oder dergleichen durch das erfindungsgemäße Verfahren frei von aktiven RNasen und/oder DNasen gemacht werden können.

Um zu prüfen, ob eine Oberfläche RNase bzw. DNase frei ist, kann eine Probe dieser Oberfläche für 1 Stunde in RNase freiem Wasser (UltraPure Distilled Water DNase/RNase Free) bzw. DNase freiem Wasser extrahiert werden. Handelt es sich bei der Oberfläche um eine innere Oberfläche eines Volumens (beispielsweise eines Polymerfolienbeutels) kann das Volumen mit RNase (bzw. DNase) freiem Wasser befüllt und für 1 Stunde extrahiert werden. 45µl dieses RNase-freien Wassers können mit einem RNase-Testkit (beispielsweise RNaseAlert^{™} Lab Test Kit, Catalog number: AM1964) oder DNase-Testkit behandelt und in einem Fluorometer (Mithras LB 940, Berthold Technologies) untersucht werden. Ist die Menge an RNase (bzw. DNase) im Wasser der Probe im Verhältnis zur Menge an RNase (bzw. DNase) im RNase/DNase-freien Wasser (Blank) unter 1,5 kann die Oberfläche als RNase (bzw. DNase) frei bezeichnet werden. Im Bereich von 1,5 bis 2,5 ist die Probe leicht kontaminiert. Ab einem Wert von 2,5 ist die Probe stark kontaminiert.

Das erfindungsgemäße Verfahren umfasst das Folgende:
- Erzeugen eines ozon-dominierten nicht thermischen Plasmas unter atmosphärischem Druck;
- Erzeugen eines stickstoff-dominierten nicht thermischen Plasmas unter atmosphärischem Druck;
- Erzeugen einer Plasmaströmung, aus dem ozon-dominierten nicht thermischen Plasma und dem stickstoff-dominierten nicht thermischen Plasma;
- Leiten der Plasmaströmung auf eine zu reinigende Oberfläche, und
- Inaktivieren von RNase und/oder DNase auf der zu reinigenden Oberfläche.

Optional kann die inaktivierte RNase und/oder DNase von der zu reinigenden Oberfläche entfernt werden, beispielsweise durch Spülung mit RNase freiem Wasser. Oftmals genügt jedoch die Inaktivierung der RNase und/oder DNase, da die Inaktivierten Enzyme die RNA/DNA nicht mehr spalten können. Weiterhin optional kann nach dem Entfernen der inaktivierten RNase und/oder DNase von der zu reinigenden Oberfläche auf diese gereinigte Oberfläche erneut Plasmaströmung geleitet werden. Diese erneute Behandlung mit Plasmaströmung erlaubt es, das Reinigungsmedium (z.B. RNase freies Wasser) von der Oberfläche zu entfernen und die Oberfläche zu trocknen.

Nichtthermisches Plasma (NTP) ist ein Plasma, das sich nicht im thermischen Gleichgewicht befindet. Die Temperaturen der enthaltenen Teilchensorten (Neutralteilchen, Ionen, Elektronen) unterscheidet sich mithin, teilweise sogar signifikant. Ozon-dominiertes nicht thermisches Plasma ist ein nicht thermisches Plasma, das vorwiegend zur Bildung von Ozon (O₃) im Plasmagas führt. Stickstoff-dominiertes nicht thermisches Plasma ist ein nicht thermisches Plasma, das vorwiegend zur Bildung von Stickstoffverbindungen, wie NO, NO₂, NOₓ, N₂O, HNO₃, HNO₂ und/oder N₂O₅ im Plasmagas führt.

Das ozon-dominierte nicht thermische Plasma kann beispielsweise mittels einer dielektrischen Barriere-Entladung oder einer Corona-Entladung erzeugt werden. Eine dielektrische Barriere-Entladung ist eine Wechselspannungs-Gasentladung, bei der mindestens eine Elektrode von einem Gasraum durch eine galvanische Trennung mittels eines Dielektrikums elektrisch isoliert ist.

Ein mit einem Prozessgas (beispielsweise Luft, insbesondere (gefilterte) Umgebungsluft) gefüllter (bzw. durchströmter) Gasraum kann durch voneinander elektrisch isolierte Elektroden ionisiert werden, um ein Plasma zu erzeugen. Hierzu wird an die Elektroden eine Wechselspannung angelegt, sodass es zur Entladung kommt. Durch Verschiebungsströme wird die Entladung auch durch die Isolation hindurch aufrechterhalten und es kann kontinuierlich elektrische Leistung in das Plasma übertragen werden.

Die Frequenz der Wechselspannung wird vorzugsweise so gewählt, dass Sie einer Resonanzfrequenz des Plasmagenerators entspricht. Die Resonanzfrequenz des Plasmagenerators hängt u.a. von Größe und Material des Plasmagenerators ab, sowie von dem eingesetzten Prozessgas. In einem Beispiel liegt die Frequenz der Wechselspannung bei ca. 40 kHz (+-10%). Je höher die Frequenz gewählt wird, desto höher ist der reaktiven Stickstoffspezies (RNS, beispielsweise NO). Je niedriger die Frequenz gewählt wird, desto höher ist der Anteil an reaktiven Sauerstoffspezies (ROS, wie Ozon). Zudem steigt mit abnehmender Frequenz die Plasmadichte.

Die Elektroden können beispielsweise als parallele Platten, als ein Drahtgeflecht auf einem Dielektrikum, eine einer Platte gegenüberliegenden Spitze und/oder dergleichen ausgeführt sein.

Eine Corona-Entladung ist eine elektrische Entladung in einem nichtleitenden Medium, beispielsweise in einem Prozessgas (beispielsweise Luft, insbesondere (gefilterte) Umgebungsluft). Die Entladung erfordert Ionen als Ladungsträger. Diese können entweder schon vorhanden sein (Plasma), oder sie bilden sich im Medium als Folge von Feldionisation, wenn die elektrische Feldstärke hoch genug ist (beispielsweise > 100 kV/m).

Das ozon-dominierte nicht thermische Plasma wird beispielsweise bei einer Leistung an einer Entladungsfläche von unterhalb 0,5 W/cm², insbesondere unterhalb von 0,4 W/cm² oder unterhalb von 0,3 W/cm², oder unterhalb von 0,2 W/cm² erzeugt wird. Ein bevorzugter Bereich zur Erzeugung von ozon-dominiertem nicht thermischem Plasma liegt zwischen 0,1 und 0,2 W/cm². Insbesondere kann die Oberflächentemperatur der Elektroden zur Erzeugung des Ozon-dominierten nicht thermischen Plasmas kleiner 90°C, insbesondere kleiner 75°C oder kleiner 60°C sein.

In einem weiteren Aspekt wird das stickstoff-dominierte nicht thermische Plasma mittels einer dielektrischen Barriere-Entladung oder einer Corona-Entladung erzeugt. Beispielswiese wird das stickstoff-dominierte nicht thermische Plasma bei einer Leistung an einer Entladungsfläche von mehr als 0,1 W/cm², insbesondere mehr als 0,2 W/cm², oder mehr als 0,5 W/cm², oder mehr als 1,5 W/cm², oder mehr als 2 W/cm² erzeugt. Ein bevorzugter Bereich liegt zwischen 0,5 und 2,2 W/cm². Insbesondere kann die Oberflächentemperatur der Elektroden zur Erzeugung des Stickstoff-dominierten nicht thermischen Plasmas größer als 90°C sein.

Als Prozessgas kann beispielsweise Luft, insbesondere getrocknete und/oder sterilisierte Luft und/oder gefilterte Umgebungsluft, Argon und/ oder dergleichen eingesetzt werden. Zudem können Mischungen von zumindest zwei unterschiedlichen Prozessgases eingesetzt werden.

Das Prozessgas kann vor der Erzeugung des Plasmas (ozon-dominiert und/oder stickstoff-dominiert) gereinigt werden, beispielsweise durch zumindest einen Filter. Für die Erzeugung des ozon-dominierten nicht thermischen Plasmas und des stickstoff-dominierten nicht thermischen Plasmas kann das gleiche Prozessgas verwendet werden (beispielsweise Luft, insbesondere (gefilterte) Umgebungsluft), oder es können unterschiedliche Prozessgase oder Mischungen verwendet werden.

Insbesondere kann die Erzeugung des ozon-dominierten nicht thermischen Plasmas und des stickstoff-dominierten nicht thermischen Plasmas simultan, vorzugsweise in einem gemeinsamen Plasmagenerator erfolgen. Hierzu kann/können die Elektrode(n) so ausgebildet sein, dass an deren Oberfläche unterschiedliche Leistungen anliegen. In einem Bereich mit niedrigerer Leistungsdichte kann beispielsweise vornehmlich ozon-dominiertes nicht-thermisches Plasma erzeugt werden und in einem Bereich mit höherer Leistungsdichte kann vornehmlich stickstoff-dominiertes nicht-thermisches Plasma erzeugt werden.

Ebenso ist es möglich, dass das ozon-dominierte nicht thermische Plasma und das stickstoff-dominierte nicht thermische Plasma seriell, d.h. in zeitlich aufeinander folgenden Abschnitten, erzeugt werden. Hierzu kann die Leistung des Plasmagenerators (periodisch) verstellt werden.

In einem weiteren Aspekt der Erfindung wird das ozon-dominierte nicht thermische Plasma in einem ersten Plasmagenerator und das stickstoff-dominierte nicht thermische Plasma in einem zweiten, vom ersten verschiedenen, Plasmagenerator erzeugt.

Das erzeugte Plasma (ozon-dominiert und stickstoff-dominiert) wird sodann zur Erzeugung einer Plasmaströmung genutzt. Das ionisierte Prozessgas wird hierzu aus dem/den Plasmagenerator(en) ausgeleitet. Das Ausleiten kann beispielsweise durch zumindest einen Lüfter und/oder zumindest eine Pumpe erfolgen. Ebenso kann der/die Plasmagenerator(en) so gestaltet sein, dass das Prozessgas unmittelbar ausgestoßen wird.

Diese Plasmaströmung, wird anschließend auf die zu reinigende Oberfläche geleitet. Die Plasmaströmung kommt folglich mit der Oberfläche in Kontakt. Um die Wirkung der Plasmaströmung zu erhöhen, kann die Plasmaströmung auf die zu reinigende Oberfläche gezielt aufgeblasen werden. Dies kann durch Plasmagasdüsen, Lüfter und/oder Pumpen erfolgen. Hierdurch kann eine besonders schnelle Inaktivierung der auf der Oberfläche befindlichen RNase/DNase erfolgen.

In einer weiteren bevorzugten Ausführungsform wird die Plasmaströmung mit einem Aerosol vermischt und auf die zu reinigenden Oberflächen geleitet. Das Aerosol kann Wasser, insbesondere destilliertes Wasser, RNase/DNase freies Wasser und/oder Plasma-aktiviertes- Wasser umfassen.

Insbesondere die Beimischung von feinen Wassertröpfchen, bevorzugt mit einer Größe von kleiner 100 µm, besonders bevorzugt mit einer Größe kleiner 80 µm führt zu einer hohen Inaktivierungsrate von RNase und/oder DNasen. Dabei wird bevorzugt destilliertes Wasser (und/oder RNase und/oder DNase freies Wasser) mit einem Ultraschallzerstäuber vernebelt und dieses Aerosolgemisch mit der Plasmaströmung (erzeugt aus Ozon-dominiertem und/oder Stickstoffdominiertem nicht thermischen Plasma vermischt und auf die zu reinigende Oberfläche geleitet.

In einer weiteren, besonders bevorzugten Ausführungsform wird Plasma-aktiviertes- Wasser (PAW) erzeugt und dieses als Aerosolgemisch auf die zu reinigende Oberfläche Fläche aufgebracht. Besonders bevorzugt wird das Aerosolgemisch mit einem Ultraschalzerstäuber erzeugt und feinste PAW-Aerosole auf die zu reinigende Oberfläche aufgebracht.

Die zu reinigende Oberfläche kann beispielsweise eine Polymeroberfläche sein, wie beispielsweise eine Oberfläche einer Polymerfolie, einer Polymertafel, oder eines sonstig geformten Polymers (spritzgegossen, extrudiert, ...). Durch die Verwendung des nicht thermischen Plasmas bzw. der Plasmaströmung wird das Polymer nicht beschädigt. Die Verwendung von ozon-dominierten und stickstoff-dominierten Plasma erlaubt eine schnelle und verlässliche Inaktivierung von RNasen und/oder DNasen.

Es sich gezeigt, dass ein Verhältnis von ozon-dominierten zu stickstoff-dominierten Plasma im Verhältnis von 70:30 (bezogen auf die Entladungsfläche) zu guten Inaktivierungsraten führt. Das NOₓ umfasst vorzugsweise NO, NO₂ und/oder höher NₓO_{y} (x ≥ 1; y ≥ 3), wobei der NO-Anteil dominiert.

Das Verhältnis der Bestandteile der Plasmaströmung kann durch die Plasmageneratorleistung, insbesondere durch die an den Generator angelegte Wechselspannung (Frequenz, Amplitude, Signalform, ...) und/oder die Generatorgeometrie bestimmt und/oder variiert werden.

Die zu reinigende Oberfläche kann eine innere Oberfläche zumindest eines Volumens umfassen. Dieses Volumen kann beispielsweise durch zumindest einen Polymerfolienbeutel oder durch einen Dekontaminationsbehälter gebildet werden. Zur Inaktivierung etwaiger RNase/DNase wird die Plasmaströmung in das Innere Oberfläche geleitet. Insbesondere kann die Plasmaströmung an zumindest einem Einlass in das Volumen geleitet werden. Zudem ist es möglich die Plasmaströmung an mehreren Einlässen in das Volumen zu leiten. Somit wird die Reinigungsleistung erhöht.

Umfasst die zu reinigende Oberfläche mehre Volumina, können diese über zumindest eine Plasmagasleitung so miteinander verbunden werden, dass die Plasmaströmung in die entsprechenden Volumina eingeleitet werden kann. Beispielsweise können mehrere Polymerfolienbeutel miteinander verbunden sein, oder mehrere Polymerfolienbeutel können parallel über einen entsprechenden Verteiler mit Plasmaströmung befüllt/durchströmt werden. Zudem ist es möglich die innere Oberfläche sogenannter Single-Use Assemblies zu reinigen. Hierzu wird die Plasmaströmung in das Single-Use Assembly eingeleitet und die innere Oberfläche gereinigt. Single-Use Assemblies umfassen typischerweise einen oder mehrere Polymerfolienbeutel sowie zumindest eine Schlauchleitung. Zudem können Ventile und/oder dergleichen Teil des Single-Use Assemblies sein. Diese Single-Use Assemblies dienen u.a. der Herstellung von Wirk- oder Impfstoffen, bzw. deren Vor- oder Zwischenprodukten.

Neben der inneren Oberfläche des Volumens können auch Gegenstände innerhalb des Volumens von aktiver RNase/DNase befreit werden. Beispielsweise können in dem Volumen Filter gehalten sein, durch die die Plasmaströmung geleitet wird. Ebenso können beispielsweise in einem Dekontaminationsbehälter weitere Gegenstände, die gereinigt werden sollen, bereitgestellt sein. Diese können beispielsweise Pipetten, Pipettenspitzen, Kanülen, Vials und/oder dergleichen umfassen. Die einströmende bzw. überströmende Plasmaströmung inaktiviert sodann RNasen und/oder DNasen.

Das Verfahren kann weiterhin eine Dichtheitsprüfung des zumindest einen Volumens umfassen, wobei als Prüfgas ein Plasmagas der erzeugten Plasmaströmung genutzt wird. Wird das Plasmagas in das Volumen eingeleitet, und der Druck in dem Volumen gemessen, kann über den Druckabfall bestimmt werden, ob das Volumen (beispielsweise ein Polymerfolienbeutel) dicht ist, oder eine Leckage aufweist. Somit kann auf eine zusätzliche Dichtheitsprüfung verzichtet werden. Unmittelbar nach der Inaktivierung der RNasen/DNasen kann somit bestimmt werden, ob das Volumen, beispielsweise ein Polymerfolienbeutel dicht und RNase/DNase frei ist. Ein Volumen, welches beide Kriterien erfüllt kann dann beispielsweise in einem Bioreaktor einer Bioprozesslinie zur Herstellung von Impf-oder Wirkstoffen, zum Transport und/oder zur Lagerung der Impf- oder Wirkstoffe eingesetzt werden. Ebenso können in den Polymerfolienbeuteln Vor- und/oder Zwischenprodukte der Impf- bzw. Wirkstoffe hergestellt, transportiert und/oder gelagert werden.

Das Verfahren kann weiterhin das Abwickeln einer Polymerfolie und/oder das Extrudieren einer Polymerfolie umfasst, und wobei die Plasmaströmung auf zumindest eine Seite der extrudierten und/oder abgewickelten Polymerfolie geleitet wird. Somit kann das Verfahren unmittelbar in den Herstellungsprozess der Folie oder in den Herstellungsprozess von Folienprodukten, wie Polymerfolienbeutel integriert werden. Ein separater Inaktivierungsschritt kann mithin entfallen, da die hergestellte Folie bzw. die hergestellten Folienprodukte unmittelbar RNase frei bzw. DNase frei sind. Ebenso ist es möglich Oberflächen mehrmals mit der Plasmaströmung zu behandeln. Beispielsweise kann die Plasmaströmung unmittelbar nach dem Abwickeln der Folie, nach dem Extrudieren der Folie, und/oder nach dem Recken (unidirektional oder bidirektional) einer Folie auf die zumindest eine Oberfläche der Folie (insbesondere eine spätere Innenseite eines Polymerfolienbeutels) geleitet werden. Hierzu kann eine Plasmagasdüse bereitgestellt sein, die vorzugsweise die gesamte Folienbreite überspannt, oder es können mehrere Plasmagasdüsen vorgesehen sein, die nebeneinander oder versetzt zueinander (in einer Abzugsrichtung der Folie) angeordnet sind, sodass die Oberfläche der Folie vorzugsweise in der gesamten Breite mit Plasmaströmung überströmt wird. Ebenso kann die zumindest eine Plasmagasdüse beweglich angeordnet sein, und über die Oberfläche der Folie bewegt werden, um die Plasmaströmung auf die zu reinigende Oberfläche zu leiten.

In einem Aspekt wird die Folie durch einen Dekontaminationsbereich geleitet, in welchem die Plasmaströmung eingeleitet wird. Der Dekontaminationsbereich kann eine Längserstreckung haben. Über eine Abzugsgeschwindigkeit der Folie kann dann die Behandlungsdauer der Oberfläche der Folie mit Plasmaströmung bestimmt werden. Innerhalb des Dekontaminationsbereichs kann die Folie (mehrfach) umgelenkt werden (z.B. mittels Rollen).

Insbesondere kann die Plasmaströmung für einen Zeitraum von zumindest 10 Minuten, oder zumindest 15 Minuten, oder zumindest 20 Minuten auf die zu reinigende Oberfläche geleitet werden.

Weiterhin kann das Verfahren das Erzeugen zumindest eines Volumens, insbesondere zumindest eines Polymerfolienbeutels, umfassen. Vorzugsweise wird das Volumen aus zumindest einer Polymerfolie hergestellt. Hierzu kann eine Polymerfolie gefaltet werden und die Randbereiche der gefalteten Polymerfolie werden miteinander (vorzugsweise stoffschlüssig) verbunden, oder mehrere Polymerfolien (zumindest zwei) werden aufeinandergelegt und miteinander zu einem Volumen, welches eine innere Oberfläche aufweist (z.B. Polymerfolienbeutel) gefügt. Das Erzeugen des zumindest einen Volumens kann ein Polymerkleben und/oder ein Polymerschweißen umfasst. Beispielsweise kann das Polymerschweißen ein Hitzeschweißen, ein Hochfrequenzscheißen, ein Impulsschweißen, ein Ultraschallschweißen, ein Reibschweißen, ein Laserschweißen und/oder dergleichen sein.

Die RNase-freie und/oder DNase-freie innere Oberfläche des Volumens kann vor und/oder nach dem Polymerkleben und/oder Polymerschweißen geschaffen werden. Beispielsweise können RNase-freie und/oder DNase-freie Folien miteinander verschweißt werden, oder es kann zunächst das Volumen hergestellt werden und sodann in das Volumen die Plasmaströmung eingeleitet werden.

Die Aufgabe wird weiterhin durch eine Vorrichtung zur Schaffung einer RNase-freien und/oder DNase-freien Oberfläche, insbesondere einer RNase-freien und/oder DNase-freien Polymeroberfläche gelöst. Die Vorrichtung ist dazu eingerichtet das vorstehend beschriebene Verfahren auszuführen. Die Vorrichtung umfasst zumindest einen Plasmareaktor zur Erzeugung eines ozon-dominierten nicht thermischen Plasmas unter atmosphärischem Druck und zumindest einen Plasmareaktor zur Erzeugung eines stickstoff-dominierten nicht thermischen Plasmas unter atmosphärischem Druck. Weiterhin umfasst die Vorrichtung zumindest eine Plasmagasleitung, welche an die Plasmareaktoren angeschlossen ist und dazu eingerichtet ist, eine aus dem ozon-dominierten nicht thermischen Plasma und dem stickstoff-dominierten nicht thermischen Plasma erzeugte Plasmaströmung auf eine zu reinigende Oberfläche zu leiten, um RNase und/oder DNase auf der zu reinigenden Oberfläche zu inaktivieren.

Der Plasmareaktor zur Erzeugung eines ozon-dominierten nicht thermischen Plasmas unter atmosphärischem Druck und der Plasmareaktor zur Erzeugung eines stickstoff-dominierten nicht thermischen Plasmas unter atmosphärischem Druck können integral ausgeführt sein. Die Erzeugung des ozon-dominierten nicht thermischen Plasmas und des stickstoff-dominierten nicht thermischen Plasmas kann an Elektroden mit unterschiedlicher Leistungsdichte, an verschiedenen Elektroden oder seriell erfolgen. Ebenso ist es möglich zwei oder mehrere verschiedene Plasmageneratoren zu nutzen.

Weiterhin kann die Plasmagasleitung dazu eingerichtet sein, die Plasmareaktoren mit zumindest einem Dekontaminationsbehälter, und/oder mit zumindest einem Volumen, welches eine innere Oberfläche aufweist, insbesondere zumindest einem Polymerfolienbeutel, und/oder mit zumindest einer Plasmagasdüse zu verbinden. Die Plasmagasdüse kann die Plasmaströmung in ein Volumen, welches eine innere Oberfläche aufweist oder direkt auf eine zu reinigende Oberfläche (z.B. auf eine abgewickelte Polymerfolie) leiten.

Insbesondere kann die Plasmagasdüse auf zumindest eine Polymerfolie gerichtet sein, wobei die Polymerfolie eine extrudierte Folie, eine abgewickelte Folie und/oder eine zu verschweißende Folie ist, und wobei die Plasmagasdüse optional beweglich eingerichtet ist.

In einem weiteren Aspekt bildet die Vorrichtung einen geschlossenen Kreislauf für die Plasmaströmung. Die erzeugte Plasmagasströmung wird also im Kreis geführt. Nach der Erzeugung wird die Plasmagasströmung auf die zu reinigende Oberfläche geleitet und anschließend nach einer optionalen Reinigung (z.B. Filterung) wieder dem/den Plasmagenerator(en) zugeführt. Somit kann die Plasmagasströmung energieeffizient erzeugt werden. Zudem kann der im Kreislauf geführten Plasmagasströmung zusätzliches Prozessgas (z.B. Luft, insbesondere (gefilterte) Umgebungsluft) zugeführt werden. Die Zuführung von Prozessgas erfolgt vorzugsweise vor dem/den Plasmagenerator(en).

Die Vorrichtung kann weiterhin eine Polymerschweißeinrichtung (z.B. eine Hitzeschweißeinrichtung, eine Ultraschallschweißeinrichtung, eine Reibschweißeinrichtung, eine Laserschweißeinrichtung, eine Hochfrequenzschweißeinrichtung, eine Impulsschweißeinrichtung und/oder dergleichen) umfassen, die zum Verschweißen von Polymerfolie zu Polymerfolienbeuteln eingerichtet ist. Die Inaktivierung kann vor oder nach dem Schweißen erfolgen.

Die Vorrichtung kann weiterhin eine Abwickelungsvorrichtung für das Abwickeln von Polymerfolie umfassen. Die Inaktivierung kann unmittelbar nach dem Abwickeln erfolgen. Zusätzlich und/oder alternativ kann die Inaktivierung auch unmittelbar nach der Herstellung der Folie, z.B. nach dem Extrudieren und/oder Recken erfolgen, d.h. bevor die Folie aufgewickelt oder weiterverarbeitet wird.

Weiterhin kann die Vorrichtung einen Dekontaminationsbehälter umfassen, der mit der zumindest einen Plasmagasleitung verbunden ist. Somit kann der Dekontaminationsbehälter und sein Inhalt von aktiver RNase/DNase befreit werden.

Weiterhin wird die Aufgabe durch einen Polymerfolienbeutel zur Verwendung in einem Bioreaktor gelöst, wobei der Polymerfolienbeutel aus einer RNase freien Polymerfolie hergestellt wurde und/oder wobei der Polymerfolienbeutel nach seiner Herstellung von aktiver RNase/DNase befreit wurde. Die innere Oberfläche des Polymerfolienbeutels ist mithin frei von RNase und/oder DNase.

### Kurze Beschreibung der Figuren

In den beigefügten Figuren ist sind spezifische Ausführungsformen der vorliegenden Erfindung dargestellt. Diese dienen dem Verständnis der Erfindung. Insbesondere zeigt
- Fig. 1: eine schematische Darstellung eines Verfahrens zur Schaffung einer RNase-freien und/oder DNase-freien Oberfläche;
- Fig. 2: eine schematische Darstellung einer ersten Vorrichtung zur Schaffung einer RNase-freien und/oder DNase-freien Oberfläche;
- Fig. 3: eine schematische Darstellung einer zweiten Vorrichtung zur Schaffung einer RNase-freien und/oder DNase-freien Oberfläche;
- Fig. 4: eine schematische Darstellung einer dritten Vorrichtung zur Schaffung einer RNase-freien und/oder DNase-freien Oberfläche;
- Fig. 5: eine schematische Darstellung einer vierten Vorrichtung zur Schaffung einer RNase-freien und/oder DNase-freien Oberfläche, und
- Fig. 6: eine schematische Darstellung einer fünften Vorrichtung zur Schaffung einer RNase-freien und/oder DNase-freien Oberfläche.

### Detaillierte Beschreibung der Figuren

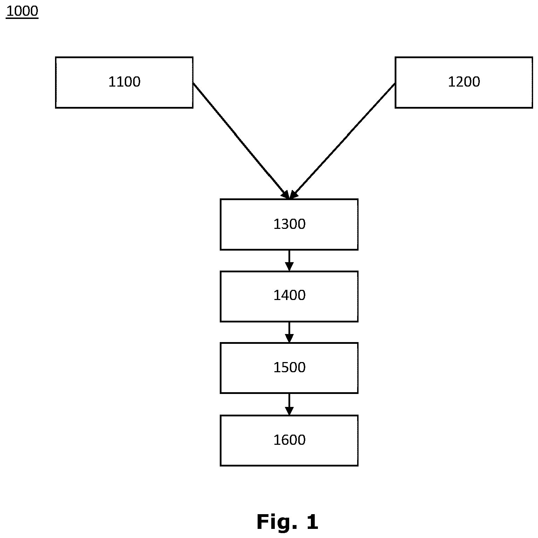

Figur 1 zeigt eine schematische Darstellung eines Verfahrens 1000 zur Schaffung einer RNase-freien und/oder DNase-freien Oberfläche. Das Verfahren 1000 umfasst Folgendes:
- Erzeugen 1100 eines ozon-dominierten nicht thermischen Plasmas unter atmosphärischem Druck;
- Erzeugen 1200 eines stickstoff-dominierten nicht thermischen Plasmas 20 unter atmosphärischem Druck;
- Erzeugen 1300 einer Plasmaströmung, aus dem ozon-dominierten nicht thermischen Plasma und dem stickstoff-dominierten nicht thermischen Plasma;
- Leiten 1400 der Plasmaströmung auf eine zu reinigende Oberfläche, und
- Inaktivieren 1500 von RNase und/oder DNase auf der zu reinigenden Oberfläche.

Optional kann die inaktivierte RNase und/oder DNase auch von der zu reinigenden Oberfläche entfernt werden 1600.

Das Verfahren 1000 wurde auf Polymerfolienbeutel mit einem Volumen von 1 Liter angewendet. Zunächst wurden die Polymerfolienbeutel mit 50 mL RNase-freiem Wasser (UltraPure Distilled Water DNase/RNase Free, Thermo Fisher Scientific) gefüllt und für zumindest 1h extrahiert. Anschließend wurden 45µl der Probe mit einem RNase-Testkit behandelt und anschließend in einem Fluorometer (Mithras LB 940, Berthold Technologies) untersucht. Das verwendete RNase-Testkit (RNaseAlert^{™} Lab Test Kit Catalog number: AM1964 der Firma Thermo Fisher Scientific) hat eine Nachweisgrenze von 3,5 x 10-7 Einheiten (~0,5 pg) RNase A. Grenzwerte, die die Begriffe RNase/DNase-frei normieren sind zum derzeitigen Zeitpunkt noch nicht festgelegt.

Es zeigte sich, dass eine längere Extraktionszeit zu einer Erhöhung des gemessenen RNase Anteils führt. Bei einer Extraktionszeit von 12 h konnte ein annähernd doppelt so hoher RNase-Anteil und bei einer Extraktionszeit von 24h ein annähernd 7,5-facher RNase-Anteil verglichen mit der einstündigen Extraktion festgestellt werden. Durch eine 20-minütige Behandlung der inneren Oberfläche der Polymerfolienbeutel gemäß dem Verfahren 1000 konnte die relative Menge an RNase im Verhältnis zu einer Probe aus reinem, RNase-freien Wasser von zunächst 11,5 auf unter 1,5 konkret auf 1,17 gesenkt werden. Die behandelte Oberfläche war somit wieder RNase frei.

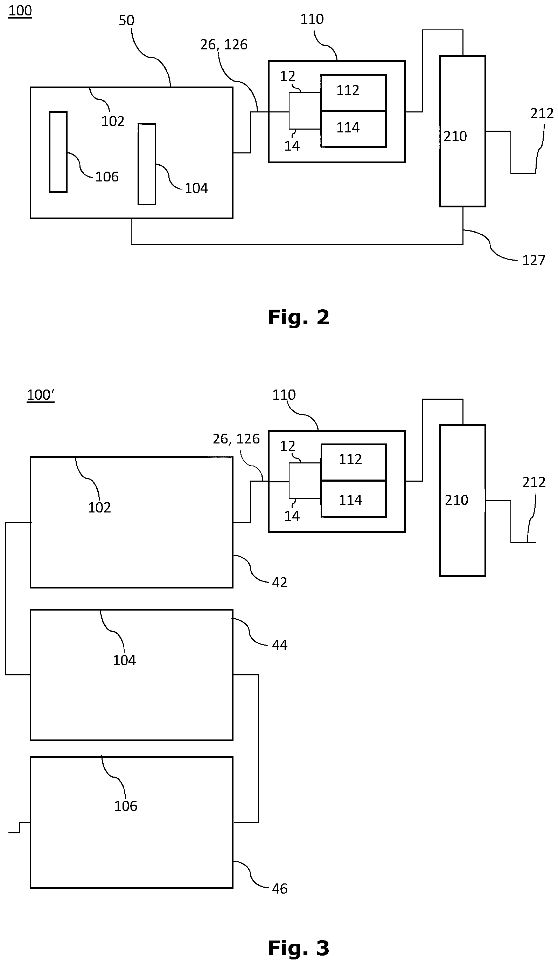

Figur 2 zeigt eine schematische Darstellung einer ersten Vorrichtung 100 zur Schaffung einer RNase-freien und/oder DNase-freien Oberfläche 102, 104, 106. Die Vorrichtung 100 umfasst zumindest einen Plasmareaktor 112 zur Erzeugung eines ozon-dominierten nicht thermischen Plasmas 12 unter atmosphärischem Druck und zumindest einen Plasmareaktor 114 zur Erzeugung eines stickstoff-dominierten nicht thermischen Plasmas 14 unter atmosphärischem Druck.

Zur Erzeugung des ozon-dominierten nicht thermischen Plasmas 12 und des stickstoff-dominierten nicht thermischen Plasmas 14 können zwei verschiedene Plasmareaktoren 112, 114 eingesetzt werden, oder es kann ein gemeinsamer Plasmareaktor 110 genutzt werden. Die Plasmareaktoren zur Erzeugung des ozon-dominierten nicht thermischen Plasmas 12 und des stickstoff-dominierten nicht thermischen Plasmas 14 können mithin integral ausgebildet sein.

Über eine Plasmagasleitung 126, welche an die Plasmareaktoren 110; 112, 114 angeschlossen ist kann eine aus dem ozon-dominierten nicht thermischen Plasma 12 und dem stickstoff-dominierten nicht thermischen Plasma 14 erzeugte Plasmaströmung 26 auf eine zu reinigende Oberfläche 102, 104, 106 geleitet werden, um RNase und/oder DNase auf der zu reinigenden Oberfläche 102, 104, 106 zu inaktivieren.

In der in Figur 2 gezeigten Ausführungsform umfasst die Vorrichtung 100 einen Dekontaminationsbehälter 50, der mit der Plasmagasleitung 126 verbunden ist. Die erzeugte Plasmaströmung 26 wird also in den Dekontaminationsbehälter 50 eingeleitet und so auf die zu reinigenden Oberflächen 102, 104, 106 geleitet. Es kann somit nicht nur die innere Oberfläche 102 des Dekontaminationsbehälters 50 von aktiver RNase/DNase befreit werden, sondern auch die Oberflächen 104, 106 der im Inneren des Dekontaminationsbehälters 50 angeordneten Gegenstände. Diese Gegenstände können beispielsweise Pipetten, Pipettenspitzen, Kanülen, Vials und/oder dergleichen umfassen.

Über eine Plasmagasleitung 127 kann die Plasmaströmung 26 in einem Kreislauf geführt werden. Nach dem Durchströmen des Dekontaminationsbehälters 50 wird die Plasmaströmung 26 beispielsweise in einem Filter 210 gereinigt und erneut an den/die Plasmareaktor(en) 110; 112, 114 übergeben. Zudem kann über einen Gaseinlass 212 weiteres Prozessgas (z.B. Luft, insbesondere (gefilterte) Umgebungsluft) in den/die Plasmareaktor(en) 110; 112, 114 geleitet werden. Das Prozessgas kann vor dem Eintritt in den/die Plasmareaktor(en) 110; 112, 114 gereinigt, insbesondere gefiltert werden.

Fig. 3 zeigt eine schematische Darstellung einer zweiten Vorrichtung 100' zur Schaffung einer RNase-freien und/oder DNase-freien Oberfläche. Die Erzeugung der Plasmaströmung erfolgt im Wesentlichen, wie in Bezug auf Figur 1 beschrieben. Die Plasmagasleitung 126 ist, wie dargestellt, jedoch nicht mit einem Dekontaminationsbehälter verbunden, sondern die Plasmaströmung 26 wird unmittelbar in einen Polymerfolienbeutel 42 eingeleitet, um dessen innere Oberfläche 102 von aktiver RNase/DNase zu befreien. Der Polymerfolienbeutel 42 kann über weitere Plasmagasleitungen (insbesondere Schläuche) mit weiteren Polymerfolienbeuteln 44, 46 verbunden sein, sodass auch deren innere Oberflächen 104, 106 als auch die damit verbundenen Bauteile (z.B. Schläuche, Ventile, Konnektoren, ...) von aktiver RNase/DNase befreit werden können. Die Polymerfolienbeutel 42, 44, 46 können, wie dargestellt in Reihe geschalten sein, oder über einen geeigneten Verteiler parallel mit Plasmaströmung 26 befüllt bzw. durchströmt werden. Auch hier kann die Plasmaströmung 26 in einem Kreislauf geführt werden.

Ebenso kann die innere Oberfläche sogenannter Single-Use Assemblies mit der Vorrichtung 100' gereinigt werden. Hierzu wird die Plasmaströmung 26 in das Single-Use Assembly eingeleitet und die innere Oberfläche gereinigt. Single-Use Assemblies umfassen typischerweise einen oder mehrere Polymerfolienbeutel 42, 44, 46 sowie zumindest eine Schlauchleitung, die die Polymerfolienbeutel verbindet. Zudem können Ventile und/oder dergleichen Teil des Single-Use Assemblies sein. Durch die Einleitung der Plasmaströmung 26 kann die innere Oberfläche eines im Wesentlichen vollständig montierten Single-Use Assemblies gereinigt werden, oder es können einzelne Teile (oder Unterbaugruppen) eines Single-Use Assemblies gereinigt werden.

Die Vorrichtungen 100 und 100' können zudem dazu eingerichtet sein, eine Dichtheitsprüfung des zumindest einen Polymerfolienbeutels 42, 44, 46 bzw. des Dekontaminationsbehälters 50 auszuführen, wobei als Prüfgas das Plasmagas der erzeugten Plasmaströmung 26 genutzt wird. Wird beispielsweise der Druck im Inneren der Polymerfolienbeutel 42, 44, 46 bzw. des Dekontaminationsbehälters 50 für einen vorbestimmten Testzeitraum konstant gehalten, kann bestimmt werden, dass die Polymerfolienbeutel/der Dekontaminationsbehälter dicht sind.

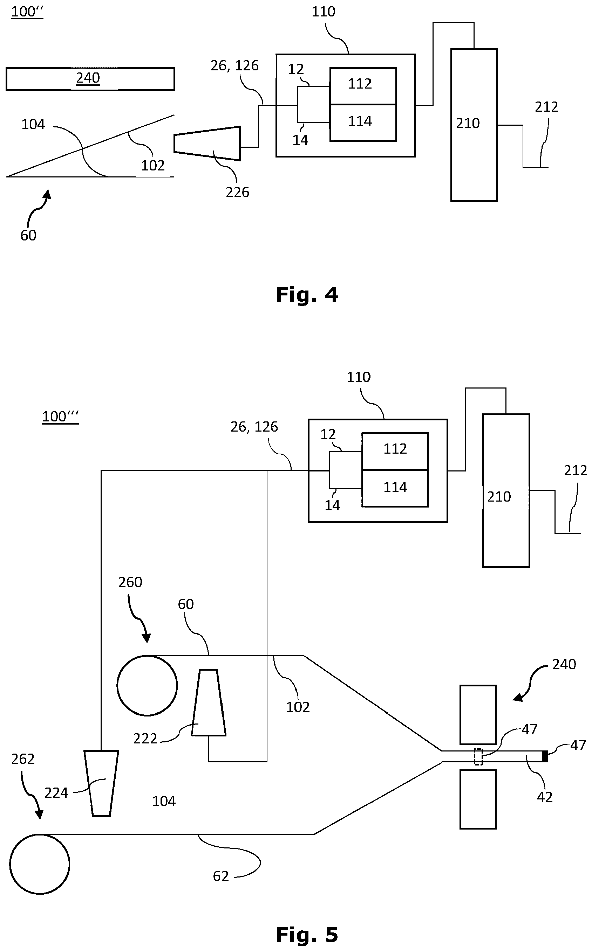

Figur 4 zeigt eine schematische Darstellung einer dritten Vorrichtung 100" zur Schaffung einer RNase-freien und/oder DNase-freien Oberfläche. Die Erzeugung der Plasmaströmung erfolgt im Wesentlichen, wie in Bezug auf Figur 1 beschrieben. Die Plasmagasleitung 126 ist, wie dargestellt, jedoch nicht mit einem Dekontaminationsbehälter verbunden, sondern mit einer Plasmagasdüse 226. Die Plasmagasdüse 226 ist auf eine Polymerfolie 60 gerichtet, die hier gefaltet vorliegt. Über eine Polymerschweißeinrichtung 240 der Vorrichtung 100" kann die gefaltete Folie zu einem Polymerfolienbeutel verschweißt werden. Die Plasmagasdüse 226 leitet die Plasmaströmung 26 vor und/oder während des Schweißens auf die spätere innere Oberfläche des Polymerfolienbeutels. Somit kann ein RNase/DNase freier Polymerfolienbeutel hergestellt werden, der beispielsweise in einem Bioreaktor als Reaktionsraum oder zu Lagerung/zum Transport von RNA/DNA-Wirk- bzw. Impfstoffen und/oder deren Vor- und/oder Zwischenprodukten eingesetzt werden kann.

Figur 5 zeigt eine schematische Darstellung einer vierten Vorrichtung 100‴ zur Schaffung einer RNase-freien und/oder DNase-freien Oberfläche. Die Erzeugung der Plasmaströmung erfolgt im Wesentlichen, wie in Bezug auf Figur 1 beschrieben. Die Plasmagasleitung 126 ist, wie dargestellt, jedoch nicht mit einem Dekontaminationsbehälter verbunden, sondern mit zwei Plasmagasdüsen 222, 224. Die erste Plasmagasdüse 222 ist auf eine Polymerfolie 60 gerichtet, die hier von einer Abwickelungsvorrichtung 260 der Vorrichtung 100‴ abgewickelt wird. Die zweite Plasmagasdüse 224 ist auf eine Polymerfolie 62 gerichtet, die von einer Abwickelungsvorrichtung 262 abgewickelt wird. Es versteht sich, dass die Vorrichtung 100‴ nicht auf zwei Plasmagasdüsen 222, 224 und zwei Abwickelungsvorrichtung 260, 262 beschränkt ist. Beispielsweise können auch mehrere Folien (zumindest drei, vier, fünf, ...) zur Polymerfolienbeutelherstellung genutzt werden, um beispielsweise dreidimensionale Polymerfolienbeutel herzustellen. Abhängig von der Anzahl der Folien, können entsprechend viele Abwickelungsvorrichtungen bereitgestellt sein. Jeder dieser Abwickelungsvorrichtungen kann zumindest eine Plasmagasdüse zugeordnet sein.

Über eine Polymerschweißeinrichtung 240 der Vorrichtung 100‴ können die erste Polymerfolie 60 und die zweite Polymerfolie 62 zu einem Polymerfolienbeutel verschweißt werden. In geeigneten Abständen (anhängig von der gewünschten Beutelgröße) kann die Polymerschweißeinrichtung 240 die Polymerfolien 60, 62 mittels einer Schweißnaht 47 verschweißen. Anstelle von zwei separaten Folien 60, 62 kann zur Polymerfolienbeutelherstellung auch ein extrudierter Folienschlauch genutzt werden.

Die Plasmagasdüse 222 leitet die Plasmaströmung 26 nach der Abwickelungsvorrichtung 260 auf eine Oberfläche der Folie 60, die später eine innere Oberfläche des Polymerfolienbeutels sein wird. Entsprechend leitet die Plasmagasdüse 224 die Plasmaströmung 26 nach der Abwickelungsvorrichtung 262 auf eine Oberfläche der Folie 62, die später eine innere Oberfläche des Polymerfolienbeutels sein wird. Somit kann ein RNase/DNase freier Polymerfolienbeutel hergestellt werden, der beispielsweise in einem Bioreaktor als Reaktionsraum oder zur Lagerung/zum Transport von RNA/DNA-Wirk- bzw. Impfstoffen und/oder deren Vor- und/oder Zwischenprodukten eingesetzt werden kann.

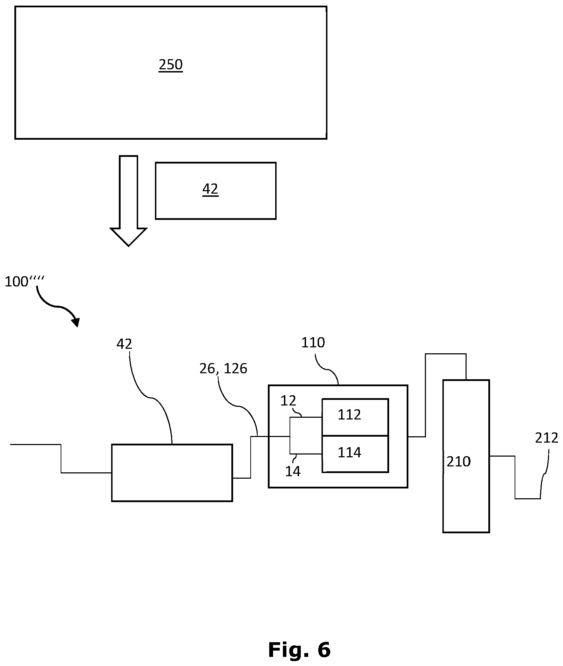

Figur 6 zeigt eine schematische Darstellung einer fünften Vorrichtung 100ʺʺ zur Schaffung einer RNase-freien und/oder DNase-freien Oberfläche. Diese Vorrichtung 100ʺʺ ist im Wesentlichen wie die Vorrichtung 100' aus Figur 3 aufgebaut. Die Vorrichtung 100ʺʺ ist einer Polymerbeutelherstellungsvorrichtung 250 nachgeschalten. Polymerfolienbeutel 42, die mittels der Polymerbeutelherstellungsvorrichtung 250 hergestellt werden, können im Anschluss an die Vorrichtung 100ʺʺ angeschlossen werden. So kann die innere Oberfläche der Polymerfolienbeutel 42 von aktiver RNase/DNase befreit werden. Die Vorrichtung 100ʺʺ kann in ein System zu Herstellung von Polymerfolienbeutel integriert sein, vorzugsweise inline, wobei die hergestellten Polymerfolienbeutel vorzugsweise automatisiert an die Vorrichtung 100ʺʺ angeschlossen werden. Die Vorrichtung 100ʺʺ kann zudem dazu eingerichtet sein, eine Dichtheitsprüfung des hergestellten Polymerfolienbeutels 42 auszuführen, wobei als Prüfgas das Plasmagas der erzeugten Plasmaströmung 26 genutzt wird.

Die Polymerbeutelherstellungsvorrichtung 250 kann zumindest eine Polymerschweißeinrichtung 240, wie sie in den Figuren 4 und 5 schematisch dargestellt ist, umfassen.

Einige der Ausführungsformen werden unter Bezugnahme auf die beigefügten Figuren ausführlicher beschrieben als andere. Andere Ausführungsformen sind jedoch ebenso in der vorliegenden Offenbarung enthalten. Die vorliegende Offenbarung sollte nicht so verstanden werden, dass sie nur auf die hier explizit dargelegten Ausführungsformen beschränkt ist; vielmehr werden diese Ausführungsformen als Beispiele angeführt, um dem Fachmann den Umfang des erfindungsgemäßen Gegenstandes zu vermitteln. Die vorliegende Erfindung kann natürlich auch auf andere Weise als die hierin dargelegte ausgeführt werden, ohne dass dadurch wesentliche Merkmale der Erfindung beeinträchtigt werden. Die vorliegenden Ausführungsformen sind in jeder Hinsicht als illustrativ und nicht einschränkend zu betrachten, sodass auch Abwandlungen der beschriebenen Ausführungsformen unter den Schutzbereich fallen können.

### Bezugszeichenliste

- 12: ozon-dominiertes nicht thermisches Plasma
- 14: stickstoff-dominiertes nicht thermisches Plasma
- 26: Plasmaströmung
- 42: Polymerfolienbeutel
- 44: Polymerfolienbeutel
- 46: Polymerfolienbeutel
- 47: Schweißnaht
- 50: Dekontaminationsbehälter
- 60: Polymerfolie
- 62: Polymerfolie
- 100: Vorrichtung
- 102: zu reinigenden Oberfläche
- 104: zu reinigenden Oberfläche
- 106: zu reinigenden Oberfläche
- 110: Plasmagenerator
- 112: Plasmagenerator
- 114: Plasmagenerator
- 126: Plasmagasleitung
- 127: Plasmagasleitung
- 210: Filter
- 212: Gaseinlass
- 222: Plasmagasdüse
- 224: Plasmagasdüse
- 226: Plasmagasdüse
- 240: Polymerschweißeinrichtung
- 250: Polymerbeutelherstellungsvorrichtung
- 260: Abwickelungsvorrichtung
- 262: Abwickelungsvorrichtung
- 1000: Verfahren
- 1100: Erzeugen eines ozon-dominierten nicht thermischen Plasmas unter atmosphärischem Druck
- 1200: Erzeugen eines stickstoff-dominierten nicht thermischen Plasmas unter atmosphärischem Druck
- 1300: Erzeugen einer Plasmaströmung, aus dem ozon-dominierten nicht thermischen Plasma und dem stickstoff-dominierten nicht thermischen Plasma
- 1400: Leiten der Plasmaströmung auf eine zu reinigende Oberfläche
- 1500: Inaktivieren von RNase und/oder DNase
- 1600: Entfernen der inaktivierten RNase und/oder DNase
- 1700: Erneutes Leiten der Plasmaströmung auf eine zu reinigende Oberfläche

## Patentansprüche

1. Verfahren (1000) zur Schaffung einer RNase-freien und/oder DNase-freien Oberfläche, insbesondere einer RNase-freien und/oder DNase-freien Polymeroberfläche, wobei das Verfahren umfasst:
Erzeugen (1100) eines ozon-dominierten nicht thermischen Plasmas (10) unter atmosphärischem Druck;
Erzeugen (1200) eines stickstoff-dominierten nicht thermischen Plasmas (20) unter atmosphärischem Druck;
Erzeugen (1300) einer Plasmaströmung (26), aus dem ozon-dominierten nicht thermischen Plasma und dem stickstoff-dominierten nicht thermischen Plasma;
Leiten (1400) der Plasmaströmung (26) auf eine zu reinigende Oberfläche (102, 104), und
Inaktivieren (1500) von RNase und/oder DNase auf der zu reinigenden Oberfläche (102, 104, 106).

2. Verfahren (1000) nach Anspruch 1, weiterhin umfassend das Entfernen (1600) der inaktivierten RNase und/oder DNase von der zu reinigenden Oberfläche (102, 104, 106), und optional
nach dem Entfernen der (1600) der inaktivierten RNase und/oder DNase von der zu reinigenden Oberfläche (102, 104, 106), Leiten (1700) der Plasmaströmung (26) auf die gereinigte Oberfläche (102, 104).

3. Verfahren (1000) nach Anspruch 1 oder 2, wobei
das ozon-dominierte nicht thermische Plasma (10) mittels einer dielektrischen Barriere-Entladung oder einer Corona-Entladung erzeugt wird, und wobei
das ozon-dominierte nicht thermische Plasma optional bei einer Leistung an einer Entladungsfläche von unterhalb 0,5 W/cm², insbesondere unterhalb von 0,4 W/cm² oder unterhalb von 0,3 W/cm², oder unterhalb von 0,2 W/cm² erzeugt wird, und/oder wobei
das stickstoff-dominierte nicht thermische Plasma (20) mittels einer dielektrischen Barriere-Entladung oder einer Corona-Entladung erzeugt wird, und wobei
das stickstoff-dominierte nicht thermische Plasma (20) optional bei einer Leistung an einer Entladungsfläche von mehr als 0,1 W/cm², insbesondere mehr als 0,2 W/cm², oder mehr als 0,5 W/cm², oder mehr als 1,5 W/cm², oder mehr als 2 W/cm² erzeugt wird, und/oder wobei
die Erzeugung (1100, 1200) des ozon-dominierten nicht thermischen Plasmas und des stickstoff-dominierten nicht thermischen Plasmas simultan, vorzugsweise in einem gemeinsamen Plasmagenerator (110) erfolgt.

4. Verfahren (1000) nach einem der Ansprüche 1 bis 3, wobei die Plasmaströmung (26) mit einem Aerosol vermischt wird und anschließend auf die zu reinigenden Oberfläche (102, 104) geleitet wird.

5. Verfahren (1000) nach einem der Ansprüche 1 bis 4, wobei
die zu reinigende Oberfläche eine innere Oberfläche zumindest eines Volumens umfasst, und wobei das zumindest eine Volumen insbesondere durch einen Polymerfolienbeutel (42, 44, 46) oder durch einen Dekontaminationsbehälter (50) gebildet wird, wobei
die Plasmaströmung (26) an zumindest einem Einlass in das Volumen geleitet wird.

6. Verfahren (1000) nach einem der Ansprüche 1 bis 5, wobei das Verfahren weiterhin eine Dichtheitsprüfung des zumindest einen Volumens umfasst, wobei als Prüfgas ein Plasmagas der erzeugten Plasmaströmung (26) genutzt wird.

7. Verfahren (1000) nach einem der Ansprüche 1 bis 6, wobei das Verfahren weiterhin das Abwickeln einer Polymerfolie (60, 62) und/oder das Extrudieren einer Polymerfolie (60, 62) umfasst, und wobei die Plasmaströmung (26) auf zumindest eine Seite der extrudierten und/oder abgewickelten Polymerfolie (60, 62) geleitet wird.

8. Verfahren (1000) nach einem der Ansprüche 1 bis 7, wobei die Plasmaströmung (26) für einen Zeitraum von zumindest 10 Minuten, oder zumindest 15 Minuten, oder zumindest 20 Minuten auf die zu reinigende Oberfläche (102, 104, 106) geleitet wird.

9. Verfahren (1000) nach einem der Ansprüche 1 bis 8, wobei das Verfahren weiterhin das
Erzeugen zumindest eines Volumens, insbesondere zumindest eines Polymerfolienbeutels (42, 44, 46), umfasst, wobei das Volumen aus zumindest einer Polymerfolie (60, 62) hergestellt wird, und das Erzeugen des zumindest eines Volumens Polymerkleben und/oder Polymerschweißen umfasst, und wobei
eine RNase-freie und/oder DNase-freie innere Oberfläche des Volumens vor und/oder nach dem Polymerkleben und/oder Polymerschweißen geschaffen wird.

10. Vorrichtung (100) zur Schaffung einer RNase-freien und/oder DNase-freien Oberfläche, insbesondere einer RNase-freien und/oder DNase-freien Polymeroberfläche, wobei die Vorrichtung (100) zum Ausführen eines Verfahrens (1000) nach einem der Ansprüche 1 bis 9 eingerichtet ist, und Folgendes umfasst:
zumindest einen Plasmareaktor (110, 112) zur Erzeugung eines ozon-dominierten nicht thermischen Plasmas (12) unter atmosphärischem Druck;
zumindest einen Plasmareaktor (110, 114) zur Erzeugung eines stickstoff-dominierten nicht thermischen Plasmas (14) unter atmosphärischem Druck;
zumindest eine Plasmagasleitung (126), welche an die Plasmareaktoren (100, 112, 114) angeschlossen ist und dazu eingerichtet ist, eine aus dem ozon-dominierten nicht thermischen Plasma und dem stickstoff-dominierten nicht thermischen Plasma erzeugte Plasmaströmung (26) auf eine zu reinigende Oberfläche (102, 104, 106) zu leiten, um RNase und/oder DNase auf der zu reinigenden Oberfläche (102, 104, 106) zu inaktivieren.

11. Vorrichtung (100) nach Anspruch 10, wobei der Plasmareaktor (110, 112) zur Erzeugung eines ozon-dominierten nicht thermischen Plasmas (12) unter atmosphärischem Druck und der Plasmareaktor (110, 114) zur Erzeugung eines stickstoff-dominierten nicht thermischen Plasmas unter atmosphärischem Druck integral ausgeführt sind.

12. Vorrichtung (100) nach Anspruch 10 oder 11, wobei die Plasmagasleitung (126) dazu eingerichtet ist die Plasmareaktoren (100, 112, 114) mit zumindest einem Dekontaminationsbehälter (50), und/oder mit zumindest einem Volumen, insbesondere zumindest einem Polymerfolienbeutel (42, 44, 46), und/oder mit zumindest einer Plasmagasdüse (222, 224, 226) zu verbinden, wobei
die Plasmagasdüse (222, 224, 226) optional auf zumindest eine Polymerfolie (60, 62) gerichtet ist, wobei die Polymerfolie (60, 62) eine extrudierte Folie, eine abgewickelte Folie und/oder eine zu verschweißende Folie ist, und wobei die Plasmagasdüse (222, 224, 226) weiter optional beweglich eingerichtet ist.

13. Vorrichtung (100) nach einem der Ansprüche 10 bis 12, wobei die Vorrichtung (100) einen geschlossenen Kreislauf für die Plasmaströmung (26) bildet.

14. Vorrichtung (100) nach einem der Ansprüche 10 bis 13, wobei
die Vorrichtung (100) eine Polymerschweißeinrichtung (240) umfasst, die zum Verschweißen von Polymerfolie (60, 62) zu Polymerfolienbeuteln (42) eingerichtet ist, und/oder wobei
die Vorrichtung (100) eine Abwickelungsvorrichtung (260, 262) für das Abwickeln von Polymerfolie (60, 62) umfasst, und/oder wobei
die Vorrichtung (100) einen Dekontaminationsbehälter (50) umfasst, der mit der zumindest einen Plasmagasleitung verbunden ist, und/oder wobei
die Vorrichtung (100) weiterhin einen Aerosolgenerator umfasst, der dazu eingerichtet ist ein Aerosol zu erzeugen, und wobei die Vorrichtung (100) dazu eingerichtet ist das erzeugte Aerosol mit der Plasmaströmung (26) zu vermischen und auf eine zu reinigende Oberfläche (102, 104, 106) zu leiten.

15. Polymerfolienbeutel (42, 44, 46) zur Verwendung in einem Bioreaktor, wobei der Polymerfolienbeutel (42, 44, 46) gemäß einem Verfahren (1000) gemäß Anspruch 9 hergestellt ist, und dessen innere Oberfläche frei von RNase und/oder DNase ist.
